# EUROPEAN PATENT SPECIFICATION

(11) **EP 2 494 954 B1**
(45) Date of publication and mention of the grant of the patent: **09.04.2014**
(21) Application number: 12157344.8
(22) Date of filing: 28.02.2012
(51) Int. Cl.: A61K 9/08, A61K 31/7032, A61P 27/02

(54) **Ophthalmic composition based on lactobionic acid useful for reducing corneal edema and inflammation**
Ophthalmische Zusammensetzung basierend auf Laktobionsäure zur Linderung von Hornhautödemen und Entzündungen
Composition ophtalmique basée sur l'acide lactobionique utile pour réduire l''dème de la cornée et l'inflammation

(30) Priority: 03.03.2011 IT RM20110105
(43) Date of publication of application: 05.09.2012
(73) Proprietor: Sooft Italia S.p.A., 63833 Montegiorgio (FM) (IT)
(72) Inventor: Cavallo, Giovanni, I-00122 Rome (IT); Stagni, Marcello, I-63822 Porto San Giorgio (Fermo) (IT); Sodo, Eugenio, I-00125 Rome (IT)
(74) Representative: Sarpi, Maurizio

(56) References cited:
- EP-A1- 1 421 974
- US-A- 4 597 965
- US-A1- 2005 171 194
- DATABASE WPI Week 200872 Thomson Scientific, London, GB; AN 2008-M13283 XP002659295, & CN 101 103 992 A (SHAANXI INST ZOOLOGY) 16 January 2008 (2008-01-16)
- INGEMANSSON ET AL: "Long-term preservation of vascular endothelium and smooth muscle", THE ANNALS OF THORACIC SURGERY, ELSEVIER, vol. 59, no. 5, 1 May 1995 (1995-05-01), pages 1177-1181, XP022231507, ISSN: 0003-4975, DOI: 10.1016/0003-4975(95)00126-6

## Description

### Field of the invention

The present invention relates to the field of topical ophthalmic preparations based on lactobionic acid that can be applied on the corneal surface in order to ensure a significant and lasting reduction both of edema and inflammatory damage of the cornea, with good local tolerance.

### Background of the invention

The cornea is a non-vascularized multilayered membrane with peculiar optical-physical properties of transparency and refraction of light.

The cornea is made up of 5 layers that, outside toward inside respectively are: corneal epithelium, Bowman's membrane, corneal stroma, Descemet's membrane, and endothelium.

The primary property of the cornea, namely its transparency, is made possible by absolute absence of blood vessels, by structural characteristics of the stroma and some essential physiological mechanisms that ensure water exchange preventing imbibition.

In physiological conditions the hydration of the cornea (in particular of the stroma) is around 78%. A stroma portion placed in distilled water absorbs water and swells, reaching hydration of 98%.

As cornea has a remarkable tendency to be impregnated of water and to swell, the epithelium and the endothelium play an important role in keeping the cornea relatively dehydrated; in fact, these two layers act as semipermeable and selective membranes able to regulate the diffusion of electrolytes and the flow of water. In addition to this role of passive barrier, the endothelium has an active transport function outwards from the parenchyma, and is able to counteract the normal hydrophilicity of the corneal tissue. It acts by a "ion pump"-type mechanism favoring the excretion of ions (Na⁺, Ca⁺⁺ bicarbonates), the hypertonia of external liquids and, consequently, the escape of water.

Alteration of these physiological processes which regulate the water exchange and assure the deturgescence, causes partial or total tissue impregnation, with consequent cornea thickening and transient or permanent loss of transparency, with final impediment to the passage of light rays and therefore loss of a good visual acuity.

This condition results in a situation of corneal edema, which is quite frequent in elderly people.

The characteristic symptoms of corneal edema comprise blurred vision and halos around lights. Because the early corneal edema symptoms are similar to those of cataract, eye examination is necessary for providing accurate diagnosis. The clinical appearance of edema varies according to the site (epithelial, stromal, endothelial) and extent of the disease process. In particular, if the dysfunction is involving endothelial cells small vesicles or bubbles occur in the cornea determining the pathological condition called bullous keratopathy.

In a healthy cornea, the endothelial cells preserve the tissue from an excessive absorption of liquids. Following a traumatic event, such as during the removal of cataract, or dystrophic material, endothelial cells may undergo death or damage. As corneal endothelial cells do normally not undergo mitotic division, a loss of cells inevitably ends in a permanent loss of the function. When the number of endothelial cells drops too much, the corneal deturgescence function is lost and excess of fluid moves in the anterior stroma and epithelium causing corneal swelling. Fluid accumulation between basal epithelium cells, determines the formation of bubbles that sometimes painfully break releasing their liquid content on the surface. This process generates typical malformations that disturb the vision and cause pain sensations.

Sometimes corneal edema can become a chronic condition, when the situation worsens, the nerve fibers are altered and are exposed, causing intense pain. In the more severe states corneal transplantation may be necessary.

Ideally, the treatment of corneal edema should be directed to the recovery of vision and elimination of the discomfort associated with the condition. To achieve these goals, clinicians may use different pharmacological or surgical approaches. The therapeutic approach to corneal edema is aimed at removing the causes of the phenomenon. When the edema is determined by a high eye pressure or by other pathology firstly the primary cause has to be resolved, for example through the application of hypotensive drugs.

A frequent cause of corneal edema is a severe inflammation of the anterior segment, as it reverses, also the edema tends to disappear.

In the specific ophthalmic field, particular interest has been focused to the preparation of the so-called "artificial tears", i.e. acqueous solutions able to simulate the function of the lachrimal fluid used to maintain and/or restore the eye physiologic lubrication. Said preparations contains certain types of polysaccharides which are not able to trigger they own synthesis by the corneal keratinocytes.
The invention described in European patent application EP 1421974 more particularly refers to the preparation of an glucan-containing ophthalmic solution, able to: increase the hydration and the lubrication of the cornea, facilitate regeneration processes, protect cornea from inflammation and having immunostimulating effects. Pharmaceutical composition for ophthalmic use for improve ocular lubrication and for promoting regenerative process according to EP 1421974 comprises: Carboxy Methyl beta-glucan (CMG) 0,5%, N-Idroxymethyl glycinate 0,001%, Sodium edetate 0,1% and Isotonic solution (pH 7,2) balancing.

The specific therapy for corneal edema is the administration of hyperosmotic eye drops able to "drain" water from the cornea to the surface (osmotherapy).

Hyperosmotic topical agents (mOsM > 1900) are ophthalmic solutions or gels designed to treat corneal edema of various origin. Removing the excess liquid, they cause a brightening up of the cornea, especially in the early stages of epithelial edema. This is not about a real pharmacological action, but an activity normalizing the tissue osmotic pressure, which is often used even immediately after surgical interventions on the eye.

Currently two types of medical devices are used:
- a hypertonic 5% sodium chloride solution (produced by Alcon and marketed with the name ADSORBONAC), which exerts a rapid effect, established within few minutes, but of short duration, about 1-2 hours; therefore, frequently repeated administration has to be taken, or
- a 35% solution of glucose, which is a thick gel, highly osmotic, helpful in cases of marked edematous component of the conjunctiva and is recommended for night use.

Such hyperosmotic preparations may be typically administered in the conjunctival sac in order to increase the osmotic pressure of tears. Following the administration of this type of preparations an osmotic gradient is established such that the extracellular fluid is hypertonic compared to intracellular one and to the other biological extracellular fluids which are separated therefrom by a semipermeable membrane (cell membrane).

The hypertonic tears drain excess water from the corneal tissue reducing the edema state. The water that comes out from the cornea due to the osmotic gradient is then drained through the lachrymal ducts.

The maximal effect is obtained when hyperosmotic preparations are administered at regular intervals and close together. The reduction of corneal edema may thus relieve ocular discomfort and irritation caused by the edema itself.

However, the use of such type of hyperosmotic topical preparations presents some important drawbacks, which consist essentially in the short duration of anti-edema effect and also by some marked discomfort (burning, temporary irritation, redness, blurred vision) at instillation. Moreover, the activity of these preparations consists only in a dehydrating action, which can be effective only if the epithelium, which acts as an imperfect semipermeable membrane, is intact. In fact, the osmotic effects occur only through an intact membrane that is able to exclude at least one of the solutes.

Patients presenting non-intact epithelium, even in the presence of serious inflammatory states, are very frequent, in such cases the aforesaid hyperosmotic preparations commercially available are poorly effective.

This lack of efficacy of hypertonic saline solutions, especially in cases of corneal edema and epithelial discontinuities, has been overcome through the formulation of artificial tears containing "mucomimetic" polymers, i.e. polymers that mimic the natural action of mucin, which in turn acts as wetting agent in the eye, and therefore pivotal for the stability of the lachrymal layer. Such polymers have been used as ophthalmic vehicles of sodium chloride at hypertonic levels. However, in these polymer solutions the osmolality of the dissolved polymer in the solution is negligibly low, and so the addition of polymers has made a minimal advantage to the usefulness of the application of hypertonic ophthalmic solutions.

The advancement of research and development in this medical-pharmaceutical field has allowed the designing of formulations for ophthalmic preparations based on high molecular weight colloids of hydrophilic polymers, such as dextran and polyacrylamide, with sufficiently high concentration to exert a colloidal osmotic pressure (oncotic pressure) equal or superior to that of a deturgescent cornea, i.e. between 40 and 200 millimeters of mercury, as described in U.S. patents 4,271,144 and U.S. patent 4,597,965.

More recently, in U.S. patent application 2009/0264375 a method for the treatment of corneal edema by means of intraocular irrigants solutions based on the precursor of keratin histidine and, optionally, of calcium glycerophosphate and/or glutathione disulfide has been described.

Despite the efforts and the attempts aimed at identifying new technical solutions in the field of ophthalmic solutions for the treatment of corneal edema, remains felt the need to realize new products able to offer better performance and further advantages compared to the described hyperosmotic solutions, especially as regard to their greater tolerance at instillation and increased duration of induced anti-edema effect.

Surprisingly, the innovative and characterizing presence of lactobionic acid in the compositions according to the present invention provides ophthalmic solutions that offer unexpected advantages compared to the hyperosmotic solutions described and employed in the treatment of corneal edema.

The lactobionic acid is the progenitor of the poly-hydroxy acids complexes, chemically it is also known with the name of aldonic acid polysaccharide, it consists of a poly-hydroxy acid, gluconic acid present in the cells as the acid form of the gluconolactone, and one sugar, i.e. galactose, natural component of glycosaminoglycans that constitutes the primary substance of the dermis.

This molecule is an excellent iron chelator and has a very powerful antioxidant action so much that for years it has been used as a main constituent of the fluids in which are preserved the organs to be transplanted. In the field of preserving donor organs, Ingemansson et al. describe the use of hyperosmotic solutions comprising lactobionic acid for preventing cell edema during hypothermic storage; however, so far no hint is given that these solutions can also be applied to living subjects to treat corneal edema (Ingemansson et al., "Long-term preservation of vascular endothelium and smooth muscle", 1995, The annals of thoracic surgery, Elsevier, 59:1177-1181).

Moreover, the lactobionic acid possesses a strong hygroscopic power for the presence of 8 hydroxyl groups. It has a very strong reparative healing action and enhances exfoliation and cell renewal by modulating the process of keratinization.

Experimental results indicate that the topical application of the lactobionic acid increases the skin turgor and corrects the histological signs of skin aging with tolerability and irritation profiles overlapping to those of physiological solution. To the many benefits of poly-hydroxy acids it has to be added the fact that these molecules have been shown to cause no sensory irritation phenomena and are therefore compatible with clinically sensitive skin, including rosacea and atopic conditions, and can be used after cosmetic treatments. For example, the US patent application 2005/171194 discloses a topical formulation, in the form of an emulsion or cream, comprising 10% lactobionic acid, 0,2% estrone or estradiol and 20% triethyl citrate (pH 3.1) for use in firming or enlarging lips, eyelids or breast.

The lactobionic acid, as well as most of the poly-hydroxy acids, has remarkable humectants and moisturizing properties, stimulates the function of the barrier of the horny layer, and therefore, stimulates the protective function of the stratum corneum, increasing the resistance of the skin to the chemical insults. The Chinese patent application CN 101 103 992 discloses antibiotic eye drops comprising 0.30% wt azithromicin, 0.29% wt lactobionic acid, 0.03% paraben, 0.22% wt sodium chloride, 0.05%wt sodium hyaluronate, 3.0% wt glycerin in an aqueous phosphate buffer solution.

In the present invention is proposed for the first time the use of lactobionic acid in eye drops useful for the care of corneal edema, also accompanied by inflammatory phenomena , that are greatly reduced thanks to its powerful antioxidant activity, and in conditions requiring healing action.

### Summary of invention

Object of the present invention are compositions useful for the treatment of corneal edema based on lactobionic acid.

Another object of the present invention are ophthalmic compositions based on lactobionic acid useful for the treatment of corneal edema in association with inflammatory phenomena.

A further object of the invention is to provide ophthalmic compositions based on lactobionic acid, characterized by a strong healing power useful for the treatment of corneal edema in conditions requiring healing activity.

Another object of the invention are ophthalmic compositions described herein comprising lactobionic acid, or its sodium salt or its potassium salt, and further comprising mannitol, and/or sodium chloride, and/or trehalose.

### Detailed description of the invention

The essential and featuring component of the ophthalmic solution according to the present invention is the lactobionic acid (4-O-beta-D-galactopyranosil-D-gluconic acid), or its sodium salt or its potassium salt. The lactobionic acid is the aldonic acid polysaccharide, consisting of a poly-hydroxy acid, gluconic acid, which in the cells is present as the acid form of the gluconolactone, and a sugar, i.e. galactose.

The concentration of lactobionic acid, or its sodium salt or its potassium salt, in the solutions according to the present invention ranges from 5% w/w to 20% w/w, preferably from 6% w/w to 16% w/w.

The solutions of the present invention may also further include other hyperosmotic agents, such as mannitol, and/or sodium chloride, and/or trehalose.

Mannitol is one of many substances that are employed for their osmotic action. Mannitol is able to exert an anti-edema effect which favors the elimination of excess fluid in the interstitial spaces of the body, in tissues and cells externally toward the blood vessels.

The mannitol (or D-(-)-mannitol), often called mannite, is a chiral alditol, with six hydroxyl groups in the six carbon atoms aliphatic chain. It is plenty naturally found in algae and fungi.

At room temperature mannitol is an odorless white solid, highly soluble in water, but practically insoluble in organic solvents.

In the food industry mannitol is widely used as a stabilizer, thickener, gelling agent and emulsifier. Furthermore, it is primarily used as a sweetener. The main problems arising from an over-intake of mannitol are borne by the gastrointestinal apparatus (laxative effect), so that in Australia is banned in baby food. The acceptable daily intake rate is about 50 mg/kg, although, apart from the unpleasant side effects, it is not dangerous to health, as being a carbohydrate, easily metabolized by human body.

Due to its osmotic properties mannitol is widely used in the pharmaceutical industry. It belongs to the class of osmotic diuretics. Parenteral injections of mannitol increases the osmotic pressure of plasma by removing water from body tissues. In particular, solutions containing mannitol in an amount ranging between 15 and 25% are intravenously injected to patients suffering from brain edema (Bhardwaj A. 2007. Curr. Neurol. Neurosci. Rep. 7, 513-521), acute renal failure (Karajala V. et al. 2009. Minerva Anestesiol. 75, 251-257), acute glaucoma or to reduce the intraocular pressure before ophthalmic surgery (Weiss D.I. et al. 1963. Arch Ophthalmol. 69, 154-158). Similar mannitol solutions are intravenously administered to force diuresis after poisoning (Schreiner G.E. et al., 1971, Trans. Am. Soc. Artif. Intern. Organs 17, 513-544), and to prepare the bowel for surgery or diagnostic procedures due to its laxative properties (Muller S. et al. 2007. Arq. Gastroenterol. 44, 244-249).

Very few descriptions of pharmaceutical compositions based on mannitol for topical application are available in the literature. The published Japanese patent document JP 9183718 describes an externally applicable composition very useful in beauty and medical care by combining a phytic acid with a specific medicinally active agent. Such medicinally active agent is selected from the group of an active oxygen remover, an antioxidant, a cell activator, an anti-inflammatory and a moisturizing agent. The active oxygen remover and antioxidant are preferably mannitol, beta-carotene and the like in an amount of 0.00001-5 wt%.

In any embodiments of the present invention, the sodium chloride has been partially, or totally, replaced by trehalose, while leaving unchanged the hypertonic character of the composition, this allows to provide less aggressive composition for the eye.

The solutions according to the present invention further comprise a buffering system to maintain the pH value in the range between 6.9 and 7.2. Several buffering systems which could be used in some embodiments according to the present invention are known to the skilled person in the art. However, in some preferred embodiments of the present invention the buffering system constituted by sodium phosphate monobasic/dibasic provides adequate buffering capacity such to maintain the pH value in the desired range.

The formulations of the present invention are prepared by mixing all the ingredients and stirring until all the components are completely dissolved. In order to achieve adequate solubilization of the lactobionic acid this should be suitably salified by addition of a base, such as NaOH, KOH, Tris, tetraborate, or other basic compounds that are easily identifiable by the experts of the relevant technical field.

Furthermore, in order to carry out the present invention it is possible to use salified lactobionic acid in the form of sodium salt and/or potassium salt which are commercially available.

The obtained solution is then sterilized by filtration (0.2 micron) or by heat treatment at 121 °C for 20 min at a 1 atmosphere pressure. However, the conditions of sterilization may be varied and optimized by experts in the field.

It is noteworthy that the product stability has been assayed at 4 °C and 40 °C. Both solutions sterilized either by filtration or heat treatment as above have resulted to be stable for nine months even at 40°C, this result allows to have a shelf life of at least 18 months at room temperature.

Therefore, in one aspect of the present invention ophthalmic compositions comprising lactobionic acid, or its sodium salt or its potassium salt, as active agent, sodium hydroxymethylglycinate and sodium phosphate buffering system are provided.

In another aspect of the present invention the ophthalmic compositions may further include mannitol and/or sodium chloride, and/or trehalose.

In some embodiments such compositions may comprise one or more additional therapeutic agents selected from the group comprising growth factors, anti-inflammatory agents, vitamins, hyaluronic acid, fibronectin, collagen, amino acids, and pharmacologically acceptable carrier substances.

The term "pharmacologically acceptable carrier substances" refers to solvents, diluents, dispersants or means which favor the suspension, isotonic agents, thickening agents and emulsifiers, preservatives, lubricants and the like, as suited to the particular dosage and forms desired according to the judgment of the formulator. Some examples of substances that act as "pharmacologically acceptable carrier substances" include, but are not limited to, sugars, starches, cellulose and its derivatives, particularly preferred in the composition according to the invention is the use of 2-hydroxyethyl cellulose as a thickening agent.

Other high molecular weight compounds which can be comprised in the formulations according to the invention are: polyvinylpyrrolidone K12 (CAS:9003-39-8), polyoxyethylene 200, non-ionic co-polymer of ethylene and propylene oxide (CAS: 9003-11-6) (marketed under the trade name Pluronic® F68).

The ophthalmic composition according to the present invention can be prepared in any pharmaceutical form which allows the application to the cornea, such as eye drops, collirium, gels, ointments, but preferably it is prepared in the form of eye drops, wherein each drop constitutes a form of dosage units in such a way to allow an easy administration and uniformity of dosage.

The eye drops according to the present invention have an osmolality which varies from a minimum value of 1500-1700 mOsM/kg and may sometimes exceed 1900 mOsM/kg.

Some examples of formulation are given below for illustrative purposes, without, however, restrict the invention.

| **Composition 1** | **%w/w** |
|---|---|
| Lactobionic acid | 16 |
| Sodium hydroxide 10% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40 ppm) |
| Distilled water | up to 100 g |

| | |
|---|---|
| Composition features: pH=7,0; osmolality=1900 mOsM/kg. | |

| **Composition 2** | **%w/w** |
|---|---|
| Sodium chloride | 2 |
| Lactobionic acid | 13 |
| Sodium hydroxide 10% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40 ppm) |
| Distilled water | up to 100 g |
| Composition features: pH=7,0; osmolality=1900 mOsM/kg. | |

| **Composition 3** | **%w/w** |
|---|---|
| Mannitol | 6 |
| Lactobionic acid | 6 |
| Sodium hydroxide 10% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40ppm) |
| Distilled water | up to 100 g |

| | |
|---|---|
| Composition features: pH=7,11; osmolality=1900mOsM/kg. | |

| **Composition 4** | **%w/w** |
|---|---|
| Sodium chloride | 2 |
| Mannitol | 6 |
| Lactobionic acid | 6 |
| Sodium hydroxide 5% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40ppm) |
| Distilled water | up to 100 g |

| | |
|---|---|
| Composition features: pH=7,1; osmolality=1850 mOsM/kg. | |

| **Composition 5** | **%w/w** |
|---|---|
| Lactobionic acid | 10 |
| Sodium hydroxide 10% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | Up to 100 g |

| | |
|---|---|
| Composition features: pH=7,0; osmolality:=1900 mOsM/kg. | |

| **Composition 6** | **%w/w** |
|---|---|
| Sodium chloride | 2 |
| Lactobionic acid | 13 |
| Sodium hydroxide 10% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | Up to 100 g |

| | |
|---|---|
| Composition features: pH=7,0; osmolality:=1900 mOsM/kg. | |

| **Composition 7** | **%w/w** |
|---|---|
| Mannitol | 6 |
| Lactobionic acid | 6 |
| Sodium hydroxide 5% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | up to 100 g |

| | |
|---|---|
| Composition features: pH=7,11; osmolality:=1900 mOsM/kg. | |

| **Composition 8** | **%w/w** |
|---|---|
| Sodium chloride | 2 |
| Mannitol | 6 |
| Lactobionic acid | 6 |
| Sodium hydroxide 10% | up to complete acid solubilization |
| Dibasic sodium phosphate | 0.6 |
| Monobasic sodium phosphate | 0.05 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | Up to 100 g |

| | |
|---|---|
| Composition features: pH=7,0; osmolality:=1900 mOsM/kg. | |

| **Composition 9** | **%w/w** |
|---|---|
| Sodium chloride | 1.5 |
| Trehalose | 5.89 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | Up to 100 g |

| | |
|---|---|
| Composition features: pH=7,1; osmolality:=1700 mOsM/kg. | |

| **Composition 10** | **%w/w** |
|---|---|
| Sodium chloride | 1.0 |
| Trehalose | 11.79 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | Up to 100 g |

| | |
|---|---|
| Composition features: pH=7,1; osmolality:=1700 mOsM/kg. | |

| **Composition 11** | **%w/w** |
|---|---|
| Sodium chloride | 0.5 |
| Trehalose | 17.68 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polpmer of ethylene | 0.02 |
| and propylene oxide | |
| Polyoxyethylene 200 | 0.5 |
| Distilled water | Up to 100 g |

| | |
|---|---|
| Composition features: pH=7,1; osmolarity:=1700 mOsM/kg. | |

| **Composition 12** | **%w/w** |
|---|---|
| Trehalose | 23.6 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Polyvinylpyrrolidone K12 | 1.670 |
| 2-Hydroxyethylcellulose | 0.450 |
| Non-ionic co-polymer of ethylene and propylene oxide | 0.02 |
| Polyoxyethylene 200 | 0.5 |
| Distilled water | Up to 100 g |

| | |
|---|---|
| Composition features: pH=7,1; osmolality:=1700 mOsM/kg. | |

In some embodiments of the present invention the above ophthalmic compositions are further comprising hydrogenated phospholipids (HPL) structured as liposomes. The so obtained formulations containing complexes of phospholipids (in form of liposomes) with sodium salt of lactobionic acid are characterized by increased ability to deliver the active principle; due to the reduced surface tension and the known capacity of liposomes to interact with ocular surfaces, also the formulation stability is increased with consequent improved delivery and bioavailability of the active principle.

Some exemplary and not limitative formulations for the ophthalmic compositions further comprising hydrogenated phospholipids (HPL) structured as liposomes are given below.

| **Composition 13** | **%w/w** |
|---|---|
| Phospholipids | 1.0 |
| Sodium chloride | 1.5 |
| Trehalose | 5.89 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Non-ionic co-polymer of ethylene and propylene oxide | 0.02 |
| Polyoxyethylene 200 | 0.5 |
| Distilled water | Up to 100 g |

| **Composition 14** | **%w/w** |
|---|---|
| Phospholipids | 1.0 |
| Sodium chloride | 1.0 |
| Trehalose | 11.79 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Non-ionic co-polymer of ethylene and propylene oxide | 0.02 |
| Polyoxyethylene 200 | 0.5 |
| Distilled water | Up to 100 g |

| **Composition 15** | **%w/w** |
|---|---|
| Phospholipids | 1.0 |
| Sodium chloride | 0.5 |
| Trehalose | 17.68 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Non-ionic co-polymer of ethylene and propylene oxide | 0.02 |
| Polyoxyethylene 200 | 0.5 |
| Distilled water | Up to 100 g |

| **Composition 16** | **%w/w** |
|---|---|
| Phospholipids | 1.0 |
| Trehalose | 23.6 |
| Sodium salt of lactobionic acid | 13.79 |
| Hydrated dibasic sodium phosphate | 0.95 |
| Hydrated monobasic sodium phosphate | 0.225 |
| Sodium hydroxymethylglycinate 1% | 0.4 ml (corresponding to 40ppm) |
| Non-ionic co-polymer of ethylene and propylene oxide | 0.02 |
| Polyoxyethylene 200 | 0.5 |
| Distilled water | Up to 100 g |

According to the present invention, the described compositions are useful in the treatment of the condition of corneal edema by topical application of a therapeutically effective amount of a composition comprising lactobionic acid as a therapeutic agent, and optionally mannitol and/or sodium chloride and/or trehalose to a subject suffering from corneal edema for a time such as to induce the desired effect. However, the strong anti-edema action of the compositions according to the invention can be applicable also to edema conditions of other body districts, such as the nasal cavities. Experiments aimed to demonstrate the therapeutic efficacy to such and alike pathological conditions are on going.

In some embodiments of the present invention, the effective therapeutic amount is that amount able to draw the excess water from the corneal tissue. Thus, according to the invention, the compositions are administered in any amount and any suitable route of administration through the eye. The exact dosage will be chosen by the physician according to the state of the patient. Dosage and administration will be adapted so as to provide sufficient levels of active ingredient or to maintain the desired effect. Additional factors that could be taken into account include the severity of the condition, course of disease, age, weight and gender of the patient, duration and frequency of administration, diet, association with other drugs, reaction sensitivities, tolerance and response to therapy.

The use of the compositions according to the present invention for the treatment of the corneal edema is not limited to humans, but its use can also be extended to all mammals.

The eye drops according to the present invention have been shown to be highly tolerable by patients, all enrolled patients declared having obtained a feeling of wellbeing through use of the product.

In order to demonstrate the effectiveness of the composition according to the present invention, the eye drops were instilled both in patients undergoing surgery on the anterior segment from the day after surgery, and in patients suffering from corneal edema due to endothelial decompensation; the administration was done in both eyes, the treated and contralateral one.

The instillation of eye drops did not cause any subjective discomfort: the absence of burning of the eyes, of feeling of foreign body, and conjunctival hyperemia have been referred by all patients, with excellent tolerability.

The biomicroscopic examination of the cornea 15 and 30 minutes after treatment, did not reveal any suffering of the corneal epithelium, thus highlighting the absence of toxic effect for the epithelium.

The use of eye drops has resulted in a significant reduction of corneal edema with excellent tolerability and compliance by the patient both in the short and long term and absence of any side effect.

The therapeutic efficacy of the compositions according to the invention, the low toxicity and the anti-inflammatory effect caused on the conjunctiva were determined by standard pharmacological procedures conducted in cell cultures or experimental animals, e.g. ED50 (therapeutic dose effective in 50% of the sample tested) and LD50 (lethal dose in 50% of the sample tested). The so obtained data were used to determine optimal dosage ranges which of the formulations above for the use of the composition in humans.

## Claims

1. Ophthalmic composition for use in the treatment of corneal edema comprising lactobionic acid, or its sodium salt or its potassium salt ranging from 5 to 20% wt, preferably from 6 to 16% wt, sodium hydroxymethylglycinate and a buffering system adapted to maintain the pH value between 6.9 and 7.2.

2. Ophthalmic composition according to claims 1 further comprising mannitol, and/or sodium chloride, and/or trehalose..

3. Ophthalmic composition according to claim 1 wherein the buffering system is constituted by sodium phosphate salts.

4. Ophthalmic composition according to any preceding claim further comprising one or more therapeutic agent selected in the group comprising growth factors, anti-inflammatory agents, vitamins, hyaluronic acid, fibronectin, collagen and amino acids.

5. Ophthalmic composition according to any preceding claim further comprising pharmaceutical acceptable carriers and excipients.

6. Ophthalmic composition according to claim 1 **characterized by** being prepared in any pharmaceutical form suitable to corneal administration, such as eye drops, collirium, gel, ointment, preferably in form of eye drops.

7. Ophthalmic composition according to claim 1 having the following formulation:
| | |
|---|---|
| Lactobionic acid | 16 %w/w |
| Sodium hydroxide 10% up to complete acid solubilization | |
| Dibasic sodium phosphate | 0.6 %w/w |
| Monobasic sodium phosphate | 0.05 %w/w |
| Sodium hydroxymethylglycinate | 40ppm |
| Distilled water | up to 100 g. |

8. Ophthalmic composition according to claim 1 having the following formulation:
| | |
|---|---|
| Sodium chloride | 2 %w/w |
| Lactobionic acid | 13 %w/w |
| Sodium hydroxide 10% up to solubilization | |
| Dibasic sodium phosphate | 0.6 %w/w |
| Monobasic sodium phosphate | 0.05 %w/w |
| Sodium hydroxymethylglycinate | 40ppm |
| Distilled water | up to 100 g. |

9. Ophthalmic composition according to claim 1 having the following formulation:
| | |
|---|---|
| Mannitol | 6 %w/w |
| Lactobionic acid | 6 %w/w |
| Sodium hydroxide 5% up to complete acid solubilization | |
| Dibasic sodium phosphate | 0.6 %w/w |
| Monobasic sodium phosphate | 0.05 %w/w |
| Sodium hydroxymethylglycinate | 40ppm |
| Distilled water | up to 100 g. |

10. Ophthalmic composition according to claim 1 having the following formulation:
| | |
|---|---|
| Sodium chloride | 2 %w/w |
| Mannitol | 6 %w/w |
| Lactobionic acid | 6 %w/w |
| Sodium hydroxide 5% up to complete acid solubilization | |
| Dibasic sodium phosphate | 0.6 %w/w |
| Monobasic sodium phosphate | 0.05 %w/w |
| Sodium hydroxymethylglycinate | 40ppm |
| Distilled water | up to 100 g. |

11. Ophthalmic composition according to claim 1 having the following formulation:
| | |
|---|---|
| Lactobionic acid | 16 %w/w |
| Sodium hydroxide 10% up to complete acid solubilization | |
| Dibasic sodium phosphate | 0.6 %w/w |
| Monobasic sodium phosphate | 0.05 %w/w |
| Sodium hydroxymethylglycinate | 40ppm |
| Polyvinylpyrrolidone K12 | 1.670 %w/w |
| 2-Hydroxyethylcellulose | 0.450 %w/w |
| Non-ionic ethylene and propylene oxide copolymer | 0.020 %w/w |
| Polyoxyethylene 200 | 0,5 %w/w |
| Distilled water | up to 100 g. |

12. Ophthalmic composition according to claim 1 having the following formulation:
| | |
|---|---|
| Sodium chloride | 2 %w/w |
| Lactobionic acid | 13 %w/w |
| Sodium hydroxide 10% up to complete acid solubilization | |
| Dibasic sodium phosphate | 0.6 %w/w |
| Monobasic sodium phosphate | 0.05 %w/w |
| Sodium hydroxymethylglycinate | 40ppm |
| Polyvinylpyrrolidone K12 | 1.670 %w/w |
| 2-Hydroxyethylcellulose | 0.450 %w/w |
| Non-ionic ethylene and propylene oxide copolymer | 0.020 %w/w |
| Polyoxyethylene 200 | 0.5 %w/w |
| Distilled water | up to 100 g. |

13. Ophthalmic composition according to claim 1 having the following formulation:
| | |
|---|---|
| Mannitol | 6 %w/w |
| Lactobionic acid | 6 %w/w |
| Sodium hydroxide 5% up to complete acid solubilization | |
| Dibasic sodium phosphate | 0.6 %w/w |
| Monobasic sodium phosphate | 0.05 %w/w |
| Polyvinylpyrrolidone K12 | 1.670 %w/w |
| 2-Hydroxyethylcellulose | 0.450 %w/w |
| Non-ionic ethylene and propylene oxide copolymer | 0.020 %w/w |
| Polyoxyethylene 200 | 0.5 %w/w |
| Sodium hydroxymethylglycinate | 40ppm |
| Distilled water | up to 100 g. |

14. Ophthalmic composition according to claim 1 having the following formulation:
| | |
|---|---|
| Sodium chloride | 2 %w/w |
| Mannitol | 6 %w/w |
| Lactobionic acid | 6 %w/w |
| Sodium hydroxide 5% up to complete acid solubilization | |
| Dibasic sodium phosphate | 0.6 %w/w |
| Monobasic sodium phosphate | 0.05 %w/w |
| Polyvinylpyrrolidone K12 | 1.670 %w/w |
| 2-Hydroxyethylcellulose | 0.450 %w/w |
| Non-ionic ethylene and propylene oxide copolymer | 0.020 %w/w |
| Polyoxyethylene 200 | 0.5 %w/w |
| Sodium hydroxymethylglycinate | 40ppm |
| Distilled water | up to 100 g. |

15. Ophthalmic composition according to claim 1 having the following formulation:
| | |
|---|---|
| Sodium chloride | 1.5 %w/w |
| Trehalose | 5.89 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Polyvinylpyrrolidone K12 | 1.670 %w/w |
| 2-Hydroxyethylcellulose | 0.450 %w/w |
| Non-ionic co-polymer of ethylene and propylene oxide | 0.02 %w/w |
| Polyoxyethylene 200 | 0.5 %w/w |
| Distilled water | Up to 100 g. |

16. Ophthalmic composition according to claim 1 having the following formulation:
| | |
|---|---|
| Sodium chloride | 1.0 %w/w |
| Trehalose | 11.79 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Polyvinylpyrrolidone K12 | 1.670 %w/w |
| 2-Hydroxyethylcellulose | 0.450 %w/w |
| Non-ionic co-polymer of ethylene and propylene oxide | 0.02 %w/w |
| Polyoxyethylene 200 | 0.5 %w/w |
| Distilled water | Up to 100 g. |

17. Ophthalmic composition according to claim 1 having the following formulation:
| | |
|---|---|
| Sodium chloride | 0.5 %w/w |
| Trehalose | 17.68 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Polyvinylpyrrolidone K12 | 1.670 %w/w |
| 2-Hydroxyethylcellulose | 0.450 %w/w |
| Non-ionic co-polymer of ethylene and propylene oxide | 0.02 %w/w |
| Polyoxyethylene 200 | 0.5 %w/w |
| Distilled water | Up to 100 g. |

18. Ophthalmic composition according to claim 1 having the following formulation:
| | |
|---|---|
| Trehalose | 23.6 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Polyvinylpyrrolidone K12 | 1.670 %w/w |
| 2-Hydroxyethylcellulose | 0.450 %w/w |
| Non-ionic co-polymer of ethylene and propylene oxide | 0.02 %w/w |
| Polyoxyethylene 200 | 0.5 %w/w |
| Distilled water | Up to 100 g. |

19. Ophthalmic composition according to claims 8-18 further comprising hydrogenated phospholipids.

20. Ophthalmic composition according to claim 1 having the following formulation:
| | |
|---|---|
| Phospholipids | 1.0 %w/w |
| Sodium chloride | 1.5 %w/w |
| Trehalose | 5.89 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Non-ionic co-polymer of ethylene and propylene oxide | 0.02 %w/w |
| Polyoxyethylene 200 | 0.5 %w/w |
| Distilled water | Up to 100 g. |

21. Ophthalmic composition according to claim 1 having the following formulation:
| | |
|---|---|
| Phospholipids | 1.0 %w/w |
| Sodium chloride | 1.0 %w/w |
| Trehalose | 11.79 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Non-ionic co-polymer of ethylene and propylene oxide | 0.02 %w/w |
| Polyoxyethylene 200 | 0.5 %w/w |
| Distilled water | Up to 100 g. |

22. Ophthalmic composition according to claim 1 having the following formulation:
| | |
|---|---|
| Phospholipids | 1.0%w/w |
| Sodium chloride | 0.5 %w/w |
| Trehalose | 17.68 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Non-ionic co-polymer of ethylene and propylene oxide | 0.02 %w/w |
| Polyoxyethylene 200 | 0.5 %w/w |
| Distilled water | Up to 100 g. |

23. Ophthalmic composition according to claim 1 having the following formulation:
| | |
|---|---|
| Phospholipids | 1.0 %w/w |
| Trehalose | 23.6 %w/w |
| Sodium salt of lactobionic acid | 13.79 %w/w |
| Hydrated dibasic sodium phosphate | 0.95 %w/w |
| Hydrated monobasic sodium phosphate | 0.225 %w/w |
| Sodium hydroxymethylglycinate | 40ppm |
| Non-ionic co-polymer of ethylene and propylene oxide | 0.02 %w/w |
| Polyoxyethylene 200 | 0.5 %w/w |
| Distilled water | Up to 100 g. |

24. Ophthalmic composition according to any preceding claim for use in the treatment of corneal edema in association to inflammation phenomena.

25. Ophthalmic composition according to any preceding claim for use in the treatment of corneal edema in conditions requiring a healing action.

## Patentansprüche

1. Ophthalmische Zusammensetzung zur Verwendung bei der Behandlung von Hornhautödem, umfassend Lactobionsäure oder deren Natriumsalz oder deren Kaliumsalz im Bereich von 5 bis 20 Gew.-%, vorzugsweise von 6 bis 16 Gew.-%, Natriumhydroxymethylglycinat und ein Puffersystem, das den pH-Wert zwischen 6,9 und 7,2 aufrechterhalten soll.

2. Ophthalmische Zusammensetzung nach Anspruch 1, des Weiteren umfassend Mannitol und/oder Natriumchlorid und/oder Trehalose.

3. Ophthalmische Zusammensetzung nach Anspruch 1, bei der das Puffersystem durch Natriumphosphatsalze gebildet wird.

4. Ophthalmische Zusammensetzung nach einem vorhergehenden Anspruch, des Weiteren umfassend ein oder mehrere Heilmittel, die in der Wachstumsfaktoren, entzündungshemmende Wirkstoffe, Vitamine, Hyaluronsäure, Fibronektin, Kollagen und Aminosäuren enthaltenden Gruppe ausgewählt werden.

5. Ophthalmische Zusammensetzung nach einem vorhergehenden Anspruch, des Weiteren umfassend pharmazeutische annehmbare Trägersubstanzen und Arzneimittelträger.

6. Ophthalmische Zusammensetzung nach Anspruch 1, **gekennzeichnet durch** Zubereitung in beliebiger pharmazeutischer Form, die geeignet ist zur Hornhautverabreichung wie etwa Augentropfen, Augenwasser, Gel, Salbe, vorzugsweise in Form von Augentropfen.

7. Ophthalmische Zusammensetzung nach Anspruch 1, die die folgende Zubereitung aufweist:
| | |
|---|---|
| Lactobionsäure | 16 Gew.-% |
| Natriumhydroxid | 10% bis zur vollständigen Säuresolubilisierung |
| Dinatriumphosphat | 0,6 Gew.-% |
| Mononatriumphosphat | 0,05 Gew.-% |
| Natriumhydroxymethylglycinat | 40 ppm |
| Destilliertes Wasser | bis zu 100 g. |

8. Ophthalmische Zusammensetzung nach Anspruch 1, die die folgende Zubereitung aufweist:
| | |
|---|---|
| Natriumchlorid | 2 Gew.-% |
| Lactobionsäure | 13 Gew.-% |
| Natriumhydroxid | 10% bis zur vollständigen Säuresolubilisierung |
| Dinatriumphosphat | 0,6 Gew.-% |
| Mononatriumphosphat | 0,05 Gew.-% |
| Natriumhydroxymethylglycinat | 40 ppm |
| Destilliertes Wasser | bis zu 100 g. |

9. Ophthalmische Zusammensetzung nach Anspruch 1, die die folgende Zubereitung aufweist:
| | |
|---|---|
| Mannitol | 6 Gew.-% |
| Lactobionsäure | 6 Gew.-% |
| Natriumhydroxid | 5% bis zur vollständigen Säuresolubilisierung |
| Dinatriumphosphat | 0,6 Gew.-% |
| Mononatriumphosphat | 0,05 Gew.-% |
| Natriumhydroxymethylglycinat | 40 ppm |
| Destilliertes Wasser | bis zu 100 g. |

10. Ophthalmische Zusammensetzung nach Anspruch 1, die die folgende Zubereitung aufweist:
| | |
|---|---|
| Natriumchlorid | 2 Gew.-% |
| Mannitol | 6 Gew.-% |
| Lactobionsäure | 6 Gew.-% |
| Natriumhydroxid | 5% bis zur vollständigen Säuresolubilisierung |
| Dinatriumphosphat | 0,6 Gew.-% |
| Mononatriumphosphat | 0,05 Gew.-% |
| Natriumhydroxymethylglycinat | 40 ppm |
| Destilliertes Wasser | bis zu 100 g. |

11. Ophthalmische Zusammensetzung nach Anspruch 1, die die folgende Zubereitung aufweist:
| | |
|---|---|
| Lactobionsäure | 16 Gew.-% |
| Natriumhydroxid | 10% bis zur vollständigen Säuresolubilisierung |
| Dinatriumphosphat | 0,6 Gew.-% |
| Mononatriumphosphat | 0,05 Gew.-% |
| Natriumhydroxymethylglycinat | 40 ppm |
| Polyvinylpyrrolidon K12 | 1,670 Gew.-% |
| 2-Hydroxyethylzellulose | 0,450 Gew.-% |
| Nichtionisches Ethylen- und Propylenoxidkopolymeres | 0,020 Gew.-% |
| Polyoxyethylen 200 | 0,5 Gew.-% |
| Destilliertes Wasser | bis zu 100 g. |

12. Ophthalmische Zusammensetzung nach Anspruch 1, die die folgende Zubereitung aufweist:
| | |
|---|---|
| Natriumchlorid | 2 Gew.-% |
| Lactobionsäure | 13 Gew.-% |
| Natriumhydroxid | 10% bis zur vollständigen Säuresolubilisierung |
| Dinatriumphosphat | 0,6 Gew.-% |
| Mononatriumphosphat | 0,05 Gew.-% |
| Natriumhydroxymethylglycinat | 40 ppm |
| Polyvinylpyrrolidon K12 | 1,670 Gew.-% |
| 2-Hydroxyethylzellulose | 0,450 Gew.-% |
| Nichtionisches Ethylen- und Propylenoxidkopolymeres | 0,020 Gew.-% |
| Polyoxyethylen 200 | 0,5 Gew.-% |
| Destilliertes Wasser | bis zu 100 g. |

13. Ophthalmische Zusammensetzung nach Anspruch 1, die die folgende Zubereitung aufweist:
| | |
|---|---|
| Mannitol | 6 Gew.-% |
| Lactobionsäure | 6 Gew.-% |
| Natriumhydroxid | 5% bis zur vollständigen Säuresolubilisierung |
| Dinatriumphosphat | 0,6 Gew.-% |
| Mononatriumphosphat | 0,05 Gew.-% |
| Polyvinylpyrrolidon K12 | 1,670 Gew.-% |
| 2-Hydroxyethylzellulose | 0,450 Gew.-% |
| Nichtionisches Ethylen- und Propylenoxidkopolymeres | 0,020 Gew.-% |
| Polyoxyethylen 200 | 0,5 Gew.-% |
| Natriumhydroxymethylglycinat | 40 ppm |
| Destilliertes Wasser | bis zu 100 g. |

14. Ophthalmische Zusammensetzung nach Anspruch 1, die die folgende Zubereitung aufweist:
| | |
|---|---|
| Natriumchlorid | 2 Gew.-% |
| Mannitol | 6 Gew.-% |
| Lactobionsäure | 6 Gew.-% |
| Natriumhydroxid | 5% bis zur vollständigen Säuresolubilisierung |
| Dinatriumphosphat | 0,6 Gew.-% |
| Mononatriumphosphat | 0,05 Gew.-% |
| Polyvinylpyrrolidon K12 | 1,670 Gew.-% |
| 2-Hydroxyethylzellulose | 0,450 Gew.-% |
| Nichtionisches Ethylen- und Propylenoxidkopolymer | 0,020 Gew.-% |
| Polyoxyethylen 200 | 0,5 Gew.-% |
| Natriumhydroxymethylglycinat | 40 ppm |
| Destilliertes Wasser | bis zu 100 g. |

15. Ophthalmische Zusammensetzung nach Anspruch 1, die die folgende Zubereitung aufweist:
| | |
|---|---|
| Natriumchlorid | 1,5 Gew.-% |
| Trehalose | 5,89 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Polyvinylpyrrolidon K12 | 1,670 Gew.-% |
| 2-Hydroxyethylzellulose | 0,450 Gew.-% |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

16. Ophthalmische Zusammensetzung nach Anspruch 1, die die folgende Zubereitung aufweist:
| | |
|---|---|
| Natriumchlorid | 1,0 Gew.-% |
| Trehalose | 11,79 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Polyvinylpyrrolidon K12 | 1,670 Gew.-% |
| 2-Hydroxyethylzellulose | 0,450 Gew.-% |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

17. Ophthalmische Zusammensetzung nach Anspruch 1, die die folgende Zubereitung aufweist:
| | |
|---|---|
| Natriumchlorid | 0,5 Gew.-% |
| Trehalose | 17,68 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Polyvinylpyrrolidon K12 | 1,670 Gew.-% |
| 2-Hydroxyethylzellulose | 0,450 Gew.-% |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

18. Ophthalmische Zusammensetzung nach Anspruch 1, die die folgende Zubereitung aufweist:
| | |
|---|---|
| Trehalose | 23,6 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Polyvinylpyrrolidon K12 | 1,670 Gew.-% |
| 2-Hydroxyethylzellulose | 0,450 Gew.-% |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

19. Ophthalmische Zusammensetzung nach den Ansprüchen 8 bis 18, des Weiteren hydrierte Phospholipide umfassend.

20. Ophthalmische Zusammensetzung nach Anspruch 1, die die folgende Zubereitung aufweist:
| | |
|---|---|
| Phospholipide | 1,0 Gew.-% |
| Natriumchlorid | 1,5 Gew.-% |
| Trehalose | 5,89 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

21. Ophthalmische Zusammensetzung nach Anspruch 1, die die folgende Zubereitung aufweist:
| | |
|---|---|
| Phospholipide | 1,0 Gew.-% |
| Natriumchlorid | 1,0 Gew.-% |
| Trehalose | 11,79 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

22. Ophthalmische Zusammensetzung nach Anspruch 1, die die folgende Zubereitung aufweist:
| | |
|---|---|
| Phospholipide | 1,0 Gew.-% |
| Natriumchlorid | 0,5 Gew.-% |
| Trehalose | 17,68 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

23. Ophthalmische Zusammensetzung nach Anspruch 1, die die folgende Zubereitung aufweist:
| | |
|---|---|
| Phospholipide | 1,0 Gew.-% |
| Trehalose | 23,6 Gew.-% |
| Natriumsalz von Lactobionsäure | 13,79 Gew.-% |
| Hydratisiertes Dinatriumphosphat | 0,95 Gew.-% |
| Hydratisiertes Mononatriumphosphat | 0,225 Gew.-% |
| Natriumhydroxymethylglycinat | 40 ppm |
| Nichtionisches Kopolymer von Ethylen- und Propylenoxid | 0,02 Gew.-% |
| Polyoxyethylen 200 | 0,5 Gew.-% |
| Destilliertes Wasser | bis zu 100 g. |

24. Ophthalmische Zusammensetzung nach einem vorgehenden Anspruch zur Verwendung bei der Behandlung von Hornhautödem im Zusammenhang mit Entzündungsvorgängen.

25. Ophthalmische Zusammensetzung nach einem vorhergehenden Anspruch zur Verwendung bei der Behandlung von Hornhautödem bei Bedingungen, die einen Heilungsprozess erfordern.

## Revendications

1. Composition ophtalmique pour l'utilisation dans le traitement de l'oedème de la cornée comprenant de l'acide lactobionique ou son sel de sodium ou son sel de potassium dans la plage de 5 à 20% en poids, de préférence de 6 à 16% en poids, de l'hydroxyméthylglycinate de sodium et un système tampon adapté pour maintenir la valeur de pH entre 6,9 et 7,2.

2. Composition ophtalmique selon la revendication 1, comprenant en outre du mannitol et/ou du chlorure de sodium et/ou du tréhalose.

3. Composition ophtalmique selon la revendication 1, dans laquelle le système tampon est constitué par des sels de phosphate de sodium.

4. Composition ophtalmique selon une quelconque revendication précédente, comprenant en outre un ou plusieurs agents thérapeutiques sélectionnés dans le groupe comprenant des facteurs de croissance, des agents anti-inflammatoires, des vitamines, de l'acide hyaluronique, la fibronectine, le collagène et des acides aminés.

5. Composition ophtalmique selon une quelconque revendication précédente, comprenant en outre des supports et excipients acceptables du point de vue pharmaceutique.

6. Composition ophtalmique selon la revendication 1, **caractérisé en ce qu'**elle est préparée sous n'importe quelle forme pharmaceutique appropriée pour l'administration cornéenne, telle que des gouttes ophtalmiques, un collyre, un gel, un onguent, de préférence sous la forme de gouttes ophtalmiques.

7. Composition ophtalmique selon la revendication 1 ayant la formulation suivante :
| | |
|---|---|
| Acide lactobionique | 16% en poids |
| Hydroxyde de sodium 10% jusqu'à la solubilisation totale de l'acide | |
| Phosphate de sodium dibasique | 0,6% en poids |
| Phosphate de sodium monobasique | 0,05% en poids |
| Hydroxyméthylglycinate de sodium | 40 ppm |
| Eau distillée | jusqu'à 100 g. |

8. Composition ophtalmique selon la revendication 1 ayant la formulation suivante :
| | |
|---|---|
| Chlorure de sodium | 2% en poids |
| Acide lactobionique | 13% en poids |
| Hydroxyde de sodium 10% jusqu'à la solubilisation totale de l'acide | |
| Phosphate de sodium dibasique | 0,6% en poids |
| Phosphate de sodium monobasique | 0,05% en poids |
| Hydroxyméthylglycinate de sodium | 40 ppm |
| Eau distillée | jusqu'à 100 g. |

9. Composition ophtalmique selon la revendication 1 ayant la formulation suivante :
| | |
|---|---|
| Mannitol | 6% en poids |
| Acide lactobionique | 6% en poids |
| Hydroxyde de sodium 5% jusqu'à la solubilisation totale de l'acide | |
| Phosphate de sodium dibasique | 0,6% en poids |
| Phosphate de sodium monobasique | 0,05% en poids |
| Hydroxyméthylglycinate de sodium | 40 ppm |
| Eau distillée | jusqu'à 100 g. |

10. Composition ophtalmique selon la revendication 1 ayant la formulation suivante :
| | |
|---|---|
| Chlorure de sodium | 2% en poids |
| Mannitol | 6% en poids |
| Acide lactobionique | 6% en poids |
| Hydroxyde de sodium 5% jusqu'à la solubilisation totale de l'acide | |
| Phosphate de sodium dibasique | 0,6% en poids |
| Phosphate de sodium monobasique | 0,05% en poids |
| Hydroxyméthylglycinate de sodium | 40 ppm |
| Eau distillée | jusqu'à 100 g. |

11. Composition ophtalmique selon la revendication 1 ayant la formulation suivante :
| | |
|---|---|
| Acide lactobionique | 16% en poids |
| Hydroxyde de sodium 10% jusqu'à la solubilisation totale de l'acide | |
| Phosphate de sodium dibasique | 0,6% en poids |
| Phosphate de sodium monobasique | 0,05% en poids |
| Hydroxyméthylglycinate de sodium | 40 ppm |
| Polyvinylpyrrolidone K12 | 1,670% en poids |
| 2-Hydroxyéthylcellulose | 0,450% en poids |
| Copolymère non ionique d'oxyde de propylène et d'éthylène | 0,020% en poids |
| Polyoxyéthylène 200 | 0,5% en poids |
| Eau distillée | jusqu'à 100 g. |

12. Composition ophtalmique selon la revendication 1 ayant la formulation suivante :
| | |
|---|---|
| Chlorure de sodium | 2% en poids |
| Acide lactobionique | 13% en poids |
| Hydroxyde de sodium 10% jusqu'à la solubilisation totale de l'acide | |
| Phosphate de sodium dibasique | 0,6% en poids |
| Phosphate de sodium monobasique | 0,05% en poids |
| Hydroxyméthylglycinate de sodium | 40 ppm |
| Polyvinylpyrrolidone K12 | 1,670% en poids |
| 2-Hydroxyéthylcellulose | 0,450% en poids |
| Copolymère non ionique d'oxyde de propylène et d'éthylène | 0,020% en poids |
| Polyoxyéthylène 200 | 0,5% en poids |
| Eau distillée | jusqu'à 100 g. |

13. Composition ophtalmique selon la revendication 1 ayant la formulation suivante :
| | |
|---|---|
| Mannitol | 6% en poids |
| Acide lactobionique | 6% en poids |
| Hydroxyde de sodium 5% jusqu'à la solubilisation totale de l'acide | |
| Phosphate de sodium dibasique | 0,6% en poids |
| Phosphate de sodium monobasique | 0,05% en poids |
| Polyvinylpyrrolidone K12 | 1,670% en poids |
| 2-Hydroxyéthylcellulose | 0,450% en poids |
| Copolymère non ionique d'oxyde de propylène et d'éthylène | 0,020% en poids |
| Polyoxyéthylène 200 | 0,5% en poids |
| Hydroxyméthylglycinate de sodium | 40 ppm |
| Eau distillée | jusqu'à 100 g. |

14. Composition ophtalmique selon la revendication 1 ayant la formulation suivante :
| | |
|---|---|
| Chlorure de sodium | 2% en poids |
| Mannitol | 6% en poids |
| Acide lactobionique | 6% en poids |
| Hydroxyde de sodium 5% jusqu'à la solubilisation totale de l'acide | |
| Phosphate de sodium dibasique | 0,6% en poids |
| Phosphate de sodium monobasique | 0,05% en poids |
| Polyvinylpyrrolidone K12 | 1,670% en poids |
| 2-Hydroxyéthylcellulose | 0,450% en poids |
| Copolymère non ionique d'oxyde de propylène et d'éthylène | 0,020% en poids |
| Polyoxyéthylène 200 | 0,5% en poids |
| Hydroxyméthylglycinate de sodium | 40 ppm |
| Eau distillée | jusqu'à 100 g. |

15. Composition ophtalmique selon la revendication 1 ayant la formulation suivante :
| | |
|---|---|
| Chlorure de sodium | 1,5% en poids |
| Tréhalose | 5,89 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Polyvinylpyrrolidone K12 | 1,670% en poids |
| 2-Hydroxyéthylcellulose | 0,450% en poids |
| Copolymère non ionique d'oxyde de propylène et d'éthylène | 0,02% en poids |
| Polyoxyéthylène 200 | 0,5% en poids |
| Eau distillée | jusqu'à 100 g. |

16. Composition ophtalmique selon la revendication 1 ayant la formulation suivante :
| | |
|---|---|
| Chlorure de sodium | 1,0% en poids |
| Tréhalose | 11,79 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Polyvinylpyrrolidone K12 | 1,670% en poids |
| 2-Hydroxyéthylcellulose | 0,450% en poids |
| Copolymère non ionique d'oxyde de propylène et d'éthylène | 0,02% en poids |
| Polyoxyéthylène 200 | 0,5% en poids |
| Eau distillée | jusqu'à 100 g. |

17. Composition ophtalmique selon la revendication 1 ayant la formulation suivante :
| | |
|---|---|
| Chlorure de sodium | 0,5% en poids |
| Tréhalose | 17,68 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Polyvinylpyrrolidone K12 | 1,670% en poids |
| 2-Hydroxyéthylcellulose | 0,450% en poids |
| Copolymère non ionique d'oxyde de propylène et d'éthylène | 0,02% en poids |
| Polyoxyéthylène 200 | 0,5% en poids |
| Eau distillée | jusqu'à 100 g. |

18. Composition ophtalmique selon la revendication 1 ayant la formulation suivante :
| | |
|---|---|
| Tréhalose | 23,6 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Polyvinylpyrrolidone K12 | 1,670% en poids |
| 2-Hydroxyéthylcellulose | 0,450% en poids |
| Copolymère non ionique d'oxyde de propylène et d'éthylène | 0,02% en poids |
| Polyoxyéthylène 200 | 0,5% en poids |
| Eau distillée | jusqu'à 100 g. |

19. Composition ophtalmique selon les revendications 8 à 18, comprenant en outre des phospholipides hydrogénés.

20. Composition ophtalmique selon la revendication 1 ayant la formulation suivante :
| | |
|---|---|
| Phospholipides | 1,0% en poids |
| Chlorure de sodium | 1,5% en poids |
| Tréhalose | 5,89 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Copolymère non ionique d'oxyde de propylène et d'éthylène | 0,02% en poids |
| Polyoxyéthylène 200 | 0,5% en poids |
| Eau distillée | jusqu'à 100 g. |

21. Composition ophtalmique selon la revendication 1 ayant la formulation suivante :
| | |
|---|---|
| Phospholipides | 1,0% en poids |
| Chlorure de sodium | 1,0% en poids |
| Tréhalose | 11,79 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Copolymère non ionique d'oxyde de propylène et d'éthylène | 0,02% en poids |
| Polyoxyéthylène 200 | 0,5% en poids |
| Eau distillée | jusqu'à 100 g. |

22. Composition ophtalmique selon la revendication 1 ayant la formulation suivante :
| | |
|---|---|
| Phospholipides | 1,0% en poids |
| Chlorure de sodium | 0,5% en poids |
| Tréhalose | 17,68 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Copolymère non ionique d'oxyde de propylène et d'éthylène | 0,02% en poids |
| Polyoxyéthylène 200 | 0,5% en poids |
| Eau distillée | jusqu'à 100 g. |

23. Composition ophtalmique selon la revendication 1 ayant la formulation suivante :
| | |
|---|---|
| Phospholipides | 1,0% en poids |
| Tréhalose | 23,6 en poids |
| Sel de sodium d'acide lactobionique | 13,79% en poids |
| Phosphate de sodium dibasique hydraté | 0,95% en poids |
| Phosphate de sodium monobasique hydraté | 0,225% en poids |
| Hydroxyméthylglycinate de sodium | 40 ppm |
| Copolymère non ionique d'oxyde de propylène et d'éthylène | 0,02% en poids |
| Polyoxyéthylène 200 | 0,5% en poids |
| Eau distillée | jusqu'à 100 g. |

24. Composition ophtalmique selon une quelconque revendication précédente, pour l'utilisation dans le traitement de l'oedème de la cornée en association avec des phénomènes d'inflammation.

25. Composition ophtalmique selon une quelconque revendication précédente, pour l'utilisation dans le traitement de l'oedème de la cornée dans des conditions nécessitant une action cicatrisante.
